# EUROPEAN PATENT APPLICATION

(11) **EP 4 681 701 A1**
(43) Date of publication of application: **21.01.2026**
(21) Application number: 24770999.1
(22) Date of filing: 15.03.2024
(51) Int. Cl.: A61K 8/81, A61K 8/02, A61K 8/25, A61K 8/26, A61K 8/73, A61Q 1/02, A61Q 1/10

(54) **AQUEOUS LIQUID COSMETIC**

(30) Priority: 16.03.2023 JP 2023042033
(71) Applicant: MITSUBISHI PENCIL COMPANY, LIMITED, Tokyo 140-8537 (JP)
(72) Inventor: YOSHIMURA, Ai, Fujioka-shi, Gunma 375-8501 (JP); SHINOHARA, Ryuichi, Fujioka-shi, Gunma 375-8501 (JP); YAMAZAKI, Yuichi, Fujioka-shi, Gunma 375-8501 (JP)
(74) Representative: Vossius & Partner Patentanwälte Rechtsanwälte mbB
(86) International application number: PCT/JP2024/010184
(87) International publication number: WO 2024/190897

(57) **Abstract**

An object of the present disclosure is to provide an aqueous liquid cosmetic that can be discharged as a direct liquid type eyeliner, eyeshadow, eyebrow product for drawing lines on eyebrows, or the like, and can be easily applied at a constant concentration without causing sedimentation of a glitter pigment even in a liquid having a low viscosity. The aqueous liquid cosmetic of the present disclosure contains at least (a) 0.1 to 10.0 mass% of a plate-shaped pigment having an average particle size of 5 to 200 µm, (b) 0.4 to 1.0 mass% of a thickener selected from clay minerals and celluloses, (c) 0.5 to 20 mass% of an acrylic polymer dispersant having a mass average molecular weight of 100000 or less and being soluble in water, and (d) water.

## Description

### Technical Field

The present specification relates to an aqueous liquid cosmetic containing a plate-shaped pigment exhibiting glittering properties, and more particularly, to an aqueous liquid cosmetic which can be suitably used for a direct liquid type eyeliner, eyeshadow, eyebrow product for drawing a line on eyebrows, and the like by suppressing sedimentation of the plate-shaped pigment.

### Background Art

In the related art, an aqueous liquid cosmetic for forming a glittering coating film on an application surface, such as skin or eyebrows, generally contains a plate-shaped pigment (glitter pigment) exhibiting glittering properties.

However, the plate-shaped pigment is liable to sediment, and once it sediments to form a hard cake in a layer form, the layered structure is difficult to peel off again and difficult to re-disperse in a liquid. A known method for solving the above problem is, for example, to prevent the formation of a hard cake of a layered plate-shaped pigment by increasing the viscosity of a vehicle, but this technique has a problem in that a discharge means and a structure thereof are limited.

On the other hand, examples of known techniques for suppressing sedimentation of plate-shaped pigments and applicators thereof include:
1) an aqueous cosmetic containing (a) a glitter pigment having an average particle size of 10 to 100 µm, (b) an acrylic resin emulsion, and (c) a thickener selected from the group consisting of silica, clay minerals, palmitoyl dipeptide, cellulose, cellulose nanofibers, and xanthan gums, in which a content of the component (c) is 0.1 to 0.35 mass%, and a viscosity at 25°C is 5 to 30 mPa·s, for the purpose of providing an aqueous cosmetic that has high glittering properties and can be used as a brush-pen type eyeliner (for example, see Patent Document 1);
2) a liquid cosmetic composition containing 0.01 to 10 mass% of a plate-shaped pigment having a surface coated with a compound selected from at least Group A (cellulose, hemicellulose, lignin, chitin, and chitosan), 0.05 to 5 mass% of layered clay mineral particles, 1 to 20 mass% of an acrylic copolymer in terms of solid content, and water, the liquid cosmetic composition being filled in an applicator equipped with an application portion having a brush, for the purpose of providing a liquid cosmetic composition that easily provides a clear glittering coating film without overlapping of plate-shaped pigments even when the liquid cosmetic composition containing a small amount of the plate-shaped pigment having glittering properties is filled in an applicator having an application portion such as a brush and applied onto an application surface such as skin (for example, see Patent Document 2); and
3) an aqueous makeup cosmetic containing (A) an aqueous alkali-thickened polymer emulsion, (B) a pigment containing at least carbon black, (C) a glittering powder, (D) an ethanol-soluble film forming agent, and (E) an alkali, for the purpose of providing an aqueous makeup cosmetic that spreads smoothly and has excellent color development and glossiness, a high cosmetic effect, good adhesion to the skin, excellent cosmetic durability, and excellent storage stability (for example, see Patent Document 3).

However, although the aqueous liquid cosmetics containing a plate-shaped pigment of Patent Documents 1 to 3 and the like are more suppressed in sedimentation of the plate-shaped pigment than in the related art, the sedimentation of the plate-shaped pigment is still insufficiently suppressed, and there are still problems such as sedimentation, insufficient discharge in a case of being loaded in a direct liquid type eyeliner, eyeshadow, or the like, and poor drawing performance (applicability).

### Citation List

### Patent Document

Patent Document 1: JP 2022-131500 A (Claims, Examples, etc.)
Patent Document 2: JP 2021-14412 A (Claims, Examples, etc.)
Patent Document 3: JP 2017-114803 A (Claims, Examples, etc.)

### Summary of Invention

### Technical Problem

The present disclosure has been made in view of the above known problems and the current situation, and aims to solve the problems, and an object thereof is to provide an aqueous liquid cosmetic that can be discharged as a direct liquid type eyeliner, eyeshadow, eyebrow product for drawing a line on eyebrows, or the like, and can be easily applied at a constant concentration without causing sedimentation of a plate-shaped pigment which is a glitter pigment even in a liquid having a low viscosity. Solution to Problem

As a result of intensive studies in view of the above-described known problems and the like, the present disclosers have found that the aqueous liquid cosmetic of the above object can be obtained by incorporating at least (a) a plate-shaped pigment having specific physical properties, a specific thickener, an acrylic polymer dispersant having specific physical properties, and water in predetermined amount ranges, and have completed the present disclosure.

That is, the aqueous liquid cosmetic of the present disclosure contains at least (a) 0.1 to 10.0 mass% of a plate-shaped pigment having an average particle size of 5 to 200 µm, (b) 0.4 to 1.0 mass% of a thickener selected from clay minerals and celluloses, (c) 0.5 to 20 mass% of an acrylic polymer dispersant having a mass average molecular weight of 100000 or less and being soluble in water, and (d) water.

The aqueous liquid cosmetic preferably has a viscosity of 10.0 mPa·s or less as measured at 25°C and a shear rate of 191.5 s⁻¹ (50 rpm). The aqueous liquid cosmetic preferably further contains an acrylic resin emulsion.

A value of a mass ratio of the (b)/the (c) is preferably 0.01 to 10. Advantageous Effects of Invention

According to the present disclosure, there is provided an aqueous liquid cosmetic that can be discharged as a direct liquid type eyeliner, eyeshadow, eyebrow product for drawing a line on eyebrows, or the like, and can be easily applied at a constant concentration without causing sedimentation of a glitter pigment even in a liquid having a low viscosity.

The object and effects of the present disclosure can be recognized and obtained especially using the components and combinations indicated in the claims. Both general explanation described above and detailed explanation described below are exemplary and explanatory and do not limit the present disclosure described in claims.

### Brief Description of Drawings

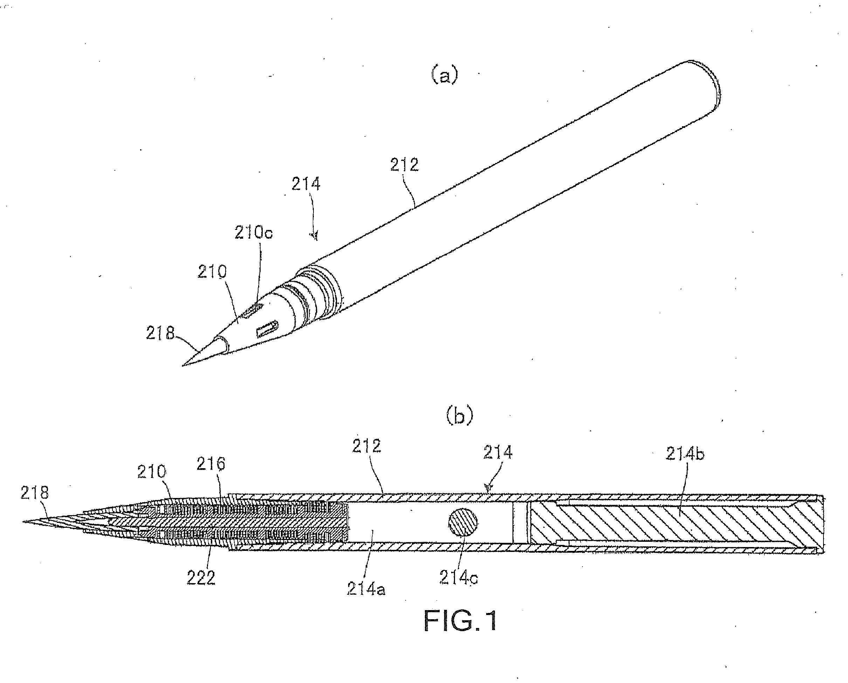

FIGs. 1(a) and 1(b) are a perspective view and a longitudinal sectional view, respectively, showing an example of an embodiment of an applicator to be filled with an aqueous liquid cosmetic of the present disclosure. Description of Embodiments

Embodiments of the present disclosure will be described below in detail. However, it should be noted that the technical scope of the present disclosure is not limited to the embodiments detailed below and includes the invention described in claims and equivalents thereof. In addition, the present disclosure can be implemented based on the contents disclosed in the present specification and technical common knowledge (including design matters and obvious matters) in the art.

The aqueous liquid cosmetic of the present disclosure contains at least (a) 0.1 to 10.0 mass% of a plate-shaped pigment having an average particle size of 5 to 200 µm, (b) 0.4 to 1.0 mass% of a thickener selected from clay minerals and celluloses, (c) 0.5 to 20 mass% of an acrylic polymer dispersant having a mass average molecular weight of 100000 or less and being soluble in water, and (d) water.

The plate-shaped pigment as the component (a) used in the present disclosure is not particularly limited as long as it is a plate-shaped pigment having an average particle size of 5 to 200 µm and having glittering properties. For example, the plate-shaped pigment is a of a pearl pigment, an aluminum flake pigment (aluminum powder pigment), a metal or metal oxide-coated glass flake, an aluminum-coated polyester film, or the like.

Generally, as an average particle size at a smooth surface of the plate-shaped pigment to be used is higher, a sparkling effect is higher. From the viewpoint of suitably exhibiting the effects of the present disclosure, the average particle size to be used is preferably in a range of 5 to 200 µm, and desirably in a range of 10 to 100 µm.

In the present disclosure (including Examples), the "average particle size" refers to a value measured and calculated by a dynamic light scattering method [particle size analyzer FPAR-1000, available from Otsuka Electronics Co., Ltd.].

When the average particle size of the plate-shaped pigment is less than 5 µm, the glittering effect is poor, and when the average particle size of the plate-shaped pigment is more than 200 µm, there is a possibility that dischargeability from a container is impaired.

Specific examples of the plate-shaped pigment as the component (a), which can be used, may include a commercially available metal oxide-coated glass flake having a trade name of "METASHINE", and a commercially available metal oxide-coated mica having a trade name of "Lumina".

Desirably, the content of the plate-shaped pigments is 0.1 to 10.0 mass% (hereinafter, "mass%" is simply referred to as "%"), preferably 0.5 to 5%, with respect to the total amount of the aqueous liquid cosmetic, for obtaining sufficient glittering properties and hiding power when the product is used.

When the content of the plate-shaped pigment is less than 0.1%, the glittering properties are poor, and when it exceeds 10.0%, the sparkling effect is naturally increased because the plate-shaped pigment is contained in a high concentration, but the cost is increased, and the applicability is likely to be lowered or to be poor when the application is advanced, which is not preferable.

As the thickener as the component (b) used in the present disclosure, at least one selected from clay minerals and celluloses is used from the viewpoint of applicability, storage stability, further suppression of sedimentation of the plate-shaped pigment described above, and the like.

Examples of the clay mineral that can be used include layered clay mineral particles, and for example, a crystalline inorganic compound having a layered structure typified by a layered silicate mineral.

The layered clay mineral particles may be natural or artificially produced. Specific examples of the layered clay mineral may include clay minerals represented by kaolinite group, smectite group, and mica group. Examples of the kaolinite group clay mineral include kaolinite. Examples of the smectite group clay mineral include montmorillonite, bentonite, saponite, hectorite, pydellite, stevensite, and nontronite. Examples of the mica group clay mineral include vermiculite, halloysite, and tetrasilicic mica. In addition to these, hydrotalcite, which is a layered double hydroxide, and the like can also be used.

Examples of commercially available products that can be used include Kunipia-F, Sumecton-SWN, and Sumecton-SA (all available from Kunimine Industries Co., Ltd.), and Ben-gel FW (all available from Hojun Co., Ltd.) .

Examples of celluloses that can be used may include crystalline cellulose, fermented cellulose, cellulose nanofiber, xanthan gum, and dextrin.

Examples of commercially available crystalline cellulose include those containing colloidal grade obtained by subjecting surfaces of fine cellulose crystals of primary particles to a coating treatment with a water-soluble polymer and the like, and specific examples thereof may include Ceolus RC-591, RCN81, RC-591NF and CL-611 and Ceolus Cream (all available from Asahi Kasei Corporation) and SunSpheres BIO SPF Booster [available from The Dow Chemical Company].

Examples of preferred thickeners as the component (b) include montmorillonite, bentonite, smectite, crystalline cellulose, fermented cellulose, and xanthan gum. More preferably, from the viewpoint of suppressing sedimentation, ensuring dischargeability from a container, and the like, a combination of layered clay mineral particles and celluloses, specifically, a combination of bentonite and fermented cellulose, a combination of bentonite and crystalline cellulose, a combination of smectite and crystalline cellulose are desirable.

The total content of these thickeners is 0.4 to 1.0%, preferably 0.4 to 0.8%, with respect to the total amount of the aqueous liquid cosmetic, from the viewpoint of ensuring dischargeability from a container while suppressing sedimentation.

When the content of the thickener is less than 0.4%, the glitter pigment sediments, and when it exceeds 1.0%, the dischargeability from a container cannot be ensured, which is not preferable.

As the acrylic polymer dispersant as the component (c) used in the present disclosure, a dispersant made of an acrylic polymer having a mass average molecular weight of 100000 or less and being soluble in water can be used, from the viewpoint of suppressing sedimentation and ensuring dischargeability from a container.

Preferably, an acrylic polymer having a mass average molecular weight of 1000 to 80000 may be indicated. Specifically, an acrylates copolymer having a mass average molecular weight as described above within a predetermined range or less, a (styrene/acrylates) copolymer, an (octylacrylamide/hydroxypropyl acrylate/butylaminoethyl methacrylate) acrylates copolymer, and the like may be indicated.

Examples of the acrylic polymer dispersant having the above characteristics and being soluble in water include commercially available products such as Luvimer 100P (available from BASF Japan), AMPHOMER HC (available from Nouryon Japan), and SYNTRAN EX149PE and SYNTRAN 5402 (both available from Interpolymer).

The content of the acrylic polymer dispersants is 0.5 to 20%, preferably 0.5 to 10% in terms of solid content with respect to the total amount of the aqueous liquid cosmetic, for ensuring dischargeability from a container while suppressing sedimentation.

When the content of the acrylic polymer dispersant is less than 0.5%, the sedimentation cannot be suppressed, and when the content exceeds 20%, the dischargeability from a container cannot be ensured, which is not preferable.

More preferably, the value of a mass ratio of the thickener as the component (b)/the acrylic polymer dispersant as the component (c) is desirably 0.01 to 10, and more preferably 0.1 to 5, from the viewpoint of suppressing sedimentation and ensuring dischargeability from a container.

In the aqueous liquid cosmetic of the present disclosure, an acrylic resin emulsion is preferably used from the viewpoint of fixability and dispersion stability.

The acrylic resin emulsion to be used includes, for example, at least one of an alkyl acrylate copolymer, an acrylates copolymer, an acrylates copolymer ammonium, an acrylic resin alkanolamine liquid, an alkyl acrylate-vinyl acetate copolymer, an (alkyl acrylate/octylacrylamide) copolymer, a silicone-modified acrylic copolymer, or an acrylic acid octylamide-acrylic acid copolymer.

In particular, the acrylic resin emulsion used in the present disclosure is preferably an acrylic alkyl resin emulsion or an acrylates copolymer from the viewpoint of further exerting the effects of the present disclosure and improving fixability.

Examples of the acrylic resin emulsion that can be used may include commercially available products such as DAITOSOL 5000STY, DAITOSOL 5000AD, DAITOSOL 5000SJ, DAITOSOL 4000SJT, DAITOSOL 5500GM, and DAITOSOL 5000PO (all available from Daito Kasei Kogyo Co., Ltd.); Yodosol GH34F and Yodosol GH800F (both available from Nouryon Japan) ; and Vinysol 1086DB, Vinysol 1087FT, Vinysol 1089HT, Vinysol 1012JC, Vinysol 1013JH, Vinysol 2140L, Vinysol 2140LH, Vinysol 1086WP, Vinysol 1087FT, and Vinysol 1089HT (all available from Daido Chemical Corporation).

The content of the acrylic resin emulsions is preferably 3 to 25%, and more preferably 5 to 20% in terms of solid content with respect to the total amount of the aqueous liquid cosmetic.

When the content of the acrylic resin emulsion is 3% or more, water resistance can be further imparted, and when the content is 25% or less, good application or the like can be performed.

In addition to the above components, the remainder of the aqueous liquid cosmetic of the present disclosure is prepared with water (purified water, ion-exchanged water, distilled water, pure water, or the like) serving as a solvent.

Furthermore, in the aqueous liquid cosmetic of the present disclosure, a humectant, an antibacterial agent, an antifoaming agent, an inorganic pigment or an organic pigment, a dye, a surfactant, a water-soluble organic solvent, and the like can be appropriately contained within a range that does not interfere with the dispersion system and does not impair the effects of the present disclosure.

Examples of the humectant that can be used include water-soluble glycols such as 1,3-butylene glycol, 1,4-butylene glycol, pentylene glycol, ethylene glycol, diethylene glycol, polyethylene glycol, propylene glycol, dipropylene glycol, and glycerin.

The content of these humectants is used in a range of preferably 1 to 30%, and more preferably 5 to 20%, with respect to the total amount of the aqueous liquid cosmetic.

Examples of the antibacterial agent that can be used include parabens, sodium dehydroacetate, phenoxyethanol, and the like. Note that, the antibacterial agent of the present disclosure includes preservatives. As parabens which are preservatives, methyl paraoxybenzoate, ethyl paraoxybenzoate, propyl paraoxybenzoate, butyl parahydroxybenzoate, isopropyl paraoxybenzoate, or the like can be used in an appropriate amount.

Examples of the antifoaming agent that can be used include polydimethylsiloxane (simethicone). This polydimethylsiloxane is a silicone oil consisting of a mixture of methylated linear siloxane polymers endblocked with trimethylsiloxy units, and, as a commercially available product, KS-66 (available from Shin-Etsu Silicone Co., Ltd.) or the like can be used in an appropriate amount.

Furthermore, in the present disclosure, for further obtaining vivid color development, a vivid color tone can be obtained by blending an organic pigment, an AL lake pigment, or a dye in addition to the plate-shaped pigment.

In the present disclosure, the pH of the aqueous liquid cosmetic is preferably in a range of 6 to 9 from the viewpoint of suppressing skin irritation. The pH can be adjusted by using a pH adjuster such as citric acid, 2-amino-2-methyl-1-propanol, 2-amino-2-methyl-1,3-propanediol, triethanolamine, L-arginine, aqueous ammonia, or sodium hydroxide. Desirably, the pH is adjusted by using, particularly preferably, citric acid or aminomethylpropanol (2-amino-2-methyl-1-propanol).

In addition, when the viscosity of the aqueous liquid cosmetic in the present disclosure is too high, the aqueous liquid cosmetic is not discharged from the brush (brush head), and when the viscosity is too low, the drawn line blurs and the aqueous liquid cosmetic is difficult to use. Therefore, the viscosity at a shear rate of 191.5 s⁻¹ (50 rpm) at 25°C is preferably in a range of 10 mPa·s or less, and more preferably in a range of 2 to 10 mPa·s.

When the viscosity is within the above range or less, a sufficient flow rate is ensured, and further, a line is easily drawn without blurring, and the aqueous liquid cosmetic is easily applied (drawn).

The viscosity range can be adjusted by combining the plate-shaped pigment, the thickener, water, and further the acrylic resin emulsion in suitable amounts.

The aqueous liquid cosmetic of the present disclosure is prepared by mixing and dispersing the above-described components in the above-described ranges of amounts and the like with a mixing and dispersing machine, for example, a bead mill, a homomixer, a disperser, an attritor, a ball mill, a sand grinder, or the like.

The aqueous liquid cosmetic of the present disclosure configured as described above can be suitably used by being stored in an applicator in which an application portion is formed of a brush (brush head). In an applicator having an application portion in which fibers are solidified with a resin or the like or an application portion of a so-called pen core type in which fibers are fused to each other, the plate-shaped pigment can be slid onto an application surface by application, but an application liquid is scraped off, and as a result, the effect of increasing the sparkling effect is reduced, and the applicability is slightly deteriorated.

Examples of the applicator that can be used include an applicator shown in FIG. 1, in which the aqueous liquid cosmetic having the above-described configuration is incorporated.

The liquid cosmetic applicator loaded with the aqueous liquid cosmetic of the present disclosure is, for example, a collector-type applicator, as illustrated in FIG. 1(a), having a barrel 214 in which a front barrel 210 and a barrel body 212 at the rear side of the front barrel 210 are fitted into each other. In the applicator, as illustrated in FIG. 1(b), a collector 216, which is formed in a comb-like shape in an embodiment in which a plurality of sheet portions are arranged in the axial direction, is disposed in the front portion of the barrel 214, and an aqueous liquid cosmetic is stored in a storage space 214a in the rear portion in the barrel 214.

The barrel body 212 at the rear portion of the barrel 214 is formed in a pipe shape that communicates with the inside and is opened in the front and rear direction. A tail plug 214b is fitted to a rear portion of the barrel body 212 that is also a rear portion of the barrel to close the rear portion of the barrel body 212. A space in the barrel 214 (also a space in the barrel body 212) sandwiched between a front end of the tail plug 214b and a rear end of the collector 216 is the storage space 214a.

In the storage space 214a, an impregnation body such as an inner cotton is not disposed, but an application liquid is directly stored, and a stirring body (e.g., ball) 214c configured to stir the application liquid is disposed.

The front barrel 210, the barrel body 212, the collector 216, and the cap may be resin molded products. A ball material made of a metal or a resin can be used for the stirring body 214c.

The collector 216 is covered and held by the front barrel 210 and the barrel body 212.

An application portion 218 formed of a brush body having a tapered shape protrudes from an opening at the front end portion of the front barrel 210, and a cap covering the application portion 218 is removably fitted to the front barrel 210. The front barrel 210 has a substantially conical side surface shape and is formed to be tapered, and the front barrel 210 is desirably formed such that the tip angle thereof is substantially equal to the tip angle of the application portion 218.

The application portion 218 is a tapered brush body (brush) made of resin fibers, natural fiber bundles, or a porous body made of resin. The rear end portion of the application portion 218 has an enlarged diameter in a flange shape. This enlarged portion is engaged with the inside of the front barrel 210 and prevents the front barrel 210 from detaching.

A bellows-like collector 216 is disposed behind the application portion 218 in the inside of the hollow tapered front barrel 210, and a core 222 penetrates through the hollow portion of the collector 216 and is disposed therein. The core 222 can be formed of a capillary member such as a resin fiber bundle, a natural fiber bundle, or a resin porous body. In the core 222, the core 222 does not protrude from the rear end portion of the collector 216 into the storage space 214a of the barrel 214 (see FIG. 1(b)). The rear end surface of the core 222 substantially matches the rear end surface of the collector 216. By matching the core 222, the rear end of the core 222 does not protrude into the storage space 214a, and the volume in the storage space 214a can be ensured. Since the rear end of the core 222 does not protrude into the storage space 214a, when the stirring body 214c is provided in the storage space 214a, the stirring body 214c does not collide with the core 222 and does not deform the core 222 even if the stirring body 214c moves in the storage space 214a. Therefore, the application liquid can sufficiently penetrate through the core.

The liquid cosmetic applicator of the above embodiment has been described with reference to, for example, a liquid cosmetic applicator of liquid eyeliner or liquid eyeshadow that is the aqueous liquid cosmetic of the present disclosure. However, the present disclosure is not limited thereto, and the present disclosure can also be applied to an eyebrow product applicator for drawing a line on eyebrows and for drawing a line on the skin.

The applicator of rotary extension type illustrated in FIG. 1 is used as a liquid pressing mechanism of the liquid cosmetic applicator of the above embodiment, but a liquid cosmetic applicator of knock extension type may be used.

The aqueous liquid cosmetic of the present disclosure configured as described above can effectively exhibit the effects of the present disclosure such as the effect that a clear glittering coating film is easily obtained without overlapping of the plate-shaped pigments even when the aqueous liquid cosmetic is filled in an applicator having an application portion such as a brush and applied onto an application surface such as skin, and the effects are presumed to be due to the following operational effects and the like.

According to the aqueous liquid cosmetic of the present disclosure, it is presumed that since the sedimentation is suppressed, the glitter pigments are prevented from overlapping in the container, and thus that the glitter pigments do not overlap even in the drawn line after application, and a high sparkling effect is exhibited. Therefore, the aqueous liquid cosmetic of the present disclosure has the above-described operational effects, and thus can be suitably used for eye makeup such as an eyeliner.

### Examples

Hereinafter, the present disclosure will be further described in detail with reference to examples and comparative examples. The present disclosure is not limited to the following examples and the like.

### Examples 1 to 11 and Comparative Examples 1 to 8

Each aqueous liquid cosmetic was prepared by using the formulation shown in Tables 1 and 2 below and by mixing and dispersing using a homomixer or disperser.

Each of the aqueous liquid cosmetics of Examples 1 to 11 and Comparative Examples 1 to 8 obtained above was evaluated for viscosity at 25°C, sparkling effect, vertical difference, and drawing performance by the following measurement methods and evaluation methods. The pH of each of these aqueous liquid cosmetics was in a range of 6.5 to 8.5.

The results are shown in Tables 1 and 2 below.

### Method of Measuring Viscosity

For each liquid cosmetic composition obtained, the viscosity at a predetermined shear rate (50 rpm: 191.5 s⁻¹) at a temperature of 25°C using a cone and plate viscometer (ELD type viscometer among TV-30 viscometers, standard cone and plate, available from Tokimec) was measured.

### Method for Evaluating Sparkling Effect

The applicator as illustrated in FIG. 1 was used to apply and spread an aqueous liquid cosmetic on the skin to evaluate the sparkling effect, the vertical difference of sparkling effect, and the drawing performance by the following test methods.

The application portion used in Examples of FIG. 1 is a tapered brush body formed of a bundle of polybutylene terephthalate resin fibers each having a diameter of 0.05 to 0.3 mm.

Each of the aqueous liquid cosmetics was applied to a skin using this applicator, and subjected to a sensory evaluation in which the sparkling effect was visually observed in accordance with the following evaluation criteria.

### Evaluation criteria:

A: The sparkling effect is high.
B: The sparkling effect is medium.
C: The sparkling effect is weak.
D: The sparkling effect is hardly observed.

### Method for Evaluating Vertical Difference of Sparkling Effect

The applicator was fixed upward and allowed to stand at room temperature (25°C) for one month. Thereafter, application was performed in the same manner as in the description of the sparkling effect, and a sensory evaluation of the sparkling effect was performed to visually observe whether or not there was a sparkling effect in accordance with the following evaluation criteria.

### Evaluation criteria:

A: The sparkling effect is high.
B: The sparkling effect is medium.
C: The sparkling effect is weak.
D: The sparkling effect is hardly observed.

### Method for Evaluating Drawing Performance

Each of the aqueous liquid cosmetics was applied to a skin using this applicator illustrated in FIG. 1, and subjected to a sensory evaluation in which the drawing performance (applicability) was visually observed in accordance with the following evaluation criteria.

### Evaluation criteria:

A: The liquid is uniformly discharged and uniformly drawn (applied) .
B: The liquid is not uniformly discharged, and a non-uniform portion is formed at the drawn (applied) portion.
C: The liquid was hardly discharged, and drawing (application) was not possible.

**[Table 1]**

| | | Example | | | | | | | | | | |
|---|---|---|---|---|---|---|---|---|---|---|---|---|
| | | 1 | 2 | 3 | 4 | 5 | 6 | 7 | 8 | 9 | 10 | 11 |
| (a) | Plate-shaped pigment A METASHINE MT1030PS *1 | 5.0 | 0.1 | 10.0 | 5.0 | 5.0 | 5.0 | 5.0 | 5.0 | 5.0 | | 5.0 |
| | Plate-shaped pigment B Frost Silver *2 | | | | | | | | | | 5.0 | |
| (b) | Layered clay mineral A bentonite *3 | 0.3 | 0.3 | 0.3 | 0.3 | 0.3 | 0.6 | 0.3 | 0.4 | | 0.3 | 0.3 |
| | Layered clay mineral B smectite *4 | | | | | | | | | 0.3 | | |
| | Cellulose crystalline cellulose *5 | 0.4 | 0.4 | 0.4 | 0.4 | 0.4 | 0.4 | 0.1 | | 0.4 | 0.4 | 0.4 |
| (c) | Acrylic polymer dispersant (styrene/acrylates) copolymer *6 | 10.0 | 10.0 | 10.0 | 0.5 | 20.0 | 10.0 | 10.0 | 10.0 | 10.0 | 10.0 | 10.0 |
| | Acrylic resin emulsion acrylates copolymer ammonium *7 | 20.0 | 20.0 | 20.0 | 20.0 | 20.0 | 20.0 | 20.0 | 20.0 | 20.0 | 20.0 | |
| | 1,3-Butylene glycol | 8.5 | 8.5 | 8.5 | 8.5 | 8.5 | 8.5 | 8.5 | 8.5 | 8.5 | 8.5 | 8.5 |
| | Preservative *8 | 4.0 | 4.0 | 4.0 | 4.0 | 4.0 | 4.0 | 4.0 | 4.0 | 4.0 | 4.0 | 4.0 |
| | pH adjusting agent (citric acid) | 0.4 | 0.4 | 0.4 | 0.4 | 0.4 | 0.4 | 0.4 | 0.4 | 0.4 | 0.4 | 0.4 |
| (d) | Water (purified water) | Balance | Balance | Balance | Balance | Balance | Balance | Balance | Balance | Balance | Balance | Balance |
| Mass ratio of (b)/(c) | | 0.07 | 0.07 | 0.07 | 1.4 | 0.035 | 0.1 | 0.04 | 0.04 | 0.07 | 0.07 | 0.07 |
| Total amount (mass%) | | 100.0 | 100.0 | 100.0 | 100.0 | 100.0 | 100.0 | 100.0 | 100.0 | 100.0 | 100.0 | 100.0 |
| Viscosity(mPa·s) of aqueous liquid cosmetic | | 5.3 | 5.4 | 5.4 | 3.7 | 8.2 | 5.5 | 4.6 | 4.2 | 5.7 | 5.3 | 4.9 |
| Evaluation | Sparkling effect | A | B | A | A | A | A | A | A | A | A | A |
| | Vertical difference | A | A | A | B | A | A | B | B | B | A | A |
| | Drawing performance | A | A | A | A | A | A | A | A | A | A | A |

**[Table 2]**

| | | Comparative Example | | | | | | | |
|---|---|---|---|---|---|---|---|---|---|
| | | 1 | 2 | 3 | 4 | 5 | 6 | 7 | 8 |
| (a) | Plate-shaped pigment A METASHINE MT1030PS *1 | 0.01 | 20.0 | 5.0 | 5.0 | 5.0 | 5.0 | 5.0 | 5.0 |
| | Plate-shaped pigment B Frost Silver *2 | | | | | | | | |
| (b) | Layered clay mineral A bentonite *3 | 0.3 | 0.3 | 0.3 | 0.3 | 0.1 | 2.0 | | |
| | Layered clay mineral B smectite *4 | | | | | | | | |
| | Cellulose crystalline cellulose *5 | 0.4 | 0.4 | 0.4 | 0.4 | | | 0.1 | 2.0 |
| (c) | Acrylic polymer dispersant (styrene/acrylates) copolymer *6 | 10.0 | 10.0 | 0.1 | 30.0 | 10.0 | 10.0 | 10.0 | 10.0 |
| | Acrylic resin emulsion acrylates copolymer ammonium *7 | 20.0 | 20.0 | 20.0 | 20.0 | 20.0 | 20.0 | 20.0 | 20.0 |
| | 1,3-Butylene glycol | 8.5 | 8.5 | 8.5 | 8.5 | 8.5 | 8.5 | 8.5 | 8.5 |
| | Preservative *8 | 4.0 | 4.0 | 4.0 | 4.0 | 4.0 | 4.0 | 4.0 | 4.0 |
| | pH adjusting agent (citric acid) | 0.4 | 0.4 | 0.4 | 0.4 | 0.4 | 0.4 | 0.4 | 0.4 |
| (d) | Water (purified water) | Balance | Balance | Balance | Balance | Balance | Balance | Balance | Balance |
| Mass ratio of (b)/(c) | | 0.07 | 0.07 | 7 | 0.02 | 0.01 | 0.2 | 0.01 | 0.2 |
| Total (mass%) | | 100.0 | 100.0 | 100.0 | 100.0 | 100.0 | 100.0 | 100.0 | 100.0 |
| Viscosity (mPa·s) | | 5.4 | 5.2 | 4.1 | 11.1 | 4.9 | 4.9 | 4.9 | 4.9 |
| Evaluation | Sparkling effect | D | A | A | A | A | A | A | A |
| | Vertical difference | A | A | C | A | D | A | D | A |
| | Drawing performance | A | C | A | C | A | C | A | C |

| | | | | | | | | | |
|---|---|---|---|---|---|---|---|---|---|
| *1 to *8 in Table 1 and 2 are as described below. *1: available from Nippon Sheet Glass Company, Ltd., average particle size of 30 µm *2: available from Toyo Aluminium K.K., average particle size of 20 µm *3: Kunipia F, available from Kunimine Industries Co., Ltd. *4: Sumecton SA, available from Kunimine Industries Co., Ltd. *5: Ceolus RC-N30, available from Asahi Kasei Corporation *6: SYNTRAN EX149PE, available from Interpolymer *7: Yodosol GH800F, available from Nouryon Japan *8: Phenoxyethanol SP, available from Yokkaichi Chemical Co., Ltd. | | | | | | | | | |

As is clear from the results of Tables 1 and 2, it is confirmed that Examples 1 to 11 supporting the present disclosure are aqueous liquid cosmetics that have no vertical difference, are excellent in sparkling effect and drawing performance, do not cause sedimentation of the glitter pigment even in a liquid having a low viscosity, and can be easily applied at a constant concentration, as compared with Comparative Examples 1 to 8 falling outside the range of the present disclosure.

### Industrial Applicability

The aqueous liquid cosmetic of the present disclosure can be suitably used for a liquid eyeliner, a liquid eyeshadow, an eyebrow product for drawing a line on eyebrows, and the like.

## Claims

1. An aqueous liquid cosmetic comprising at least (a) 0.1 to 10.0 mass% of a plate-shaped pigment having an average particle size of 5 to 200 µm, (b) 0.4 to 1.0 mass% of a thickener selected from clay minerals and celluloses, (c) 0.5 to 20 mass% of an acrylic polymer dispersant having a mass average molecular weight of 100000 or less and being soluble in water, and (d) water.

2. The aqueous liquid cosmetic according to claim 1, wherein a viscosity as measured at 25°C and a shear rate of 191.5 s⁻¹ (50 rpm) is 10.0 mPa·s or less.

3. The aqueous liquid cosmetic according to claim 1 or 2, further comprising an acrylic resin emulsion.

4. The aqueous liquid cosmetic according to claim 1 or 2, wherein a value of a mass ratio of the (b)/the (c) is 0.01 to 10.
